# EUROPÄISCHE PATENTANMELDUNG

(11) **EP 0 677 586 A1**
(43) Veröffentlichungstag der Anmeldung: **18.10.1995**
(21) Anmeldenummer: 95103015.4
(22) Anmeldetag: 03.03.1995
(51) Int. Cl.: C12N 15/57, C12N 9/52, A61K 38/44

(54) **Rekombinante Collagenase Typ II aus Clostridium histolyticum und ihre Verwendung zur Isolierung von Zellen und Zellverbänden**

(30) Priorität: 16.03.1994 DE 4408940
(71) Anmelder: BOEHRINGER MANNHEIM GMBH, D-68305 Mannheim (DE)
(72) Erfinder: Ambrosius, Dorothee, Dr., D-81477 München (DE); Hesse, Friederike, Dr., D-80802 München (DE); Burtscher, Helmut, Dr., D-82392 Habach (DE)

(57) **Zusammenfassung**

Verfahren zur Auflösung von Zellgewebe und Freisetzung von darin enthaltenen Zellen oder Zellverbänden durch Inkubation des Zellgewebes mit einer Collagenase Class II aus Clostridium histolyticum, welche nach 12 Einfrier-/Auftauzyklen noch in beträchtlichem Umfang Collagenase-Aktivität besitzt und in einem charakteristischen Teilbereich SEQ ID NO:2 oder 10 enthält oder in diesem Bereich von SEQ ID NO:1, SEQ ID NO: 9 oder einer Sequenz, welche im Rahmen der Degeneration des genetischen Codes für die gleiche Aminosäuresequenz codiert wird und das Produkt einer prokaryontischen oder eukaryontischen Expression einer exogenen DNA ist, bis zur Freisetzung der Zellen oder Zellverbände im gewünschten Umfang und Abtrennung der Zellen oder der Zellverbände von den Zellgewebeanteilen.

SEQUENZPROTOKOLL

(1) ALLGEMEINE ANGABEN:

(i) ANMELDER:
(A) NAME: BOEHRINGER MANNHEIM GMBH
(B) STRASSE: Sandhofer Str. 116
(C) ORT: Mannheim
(E) LAND: Germany
(F) POSTLEITZAHL: D-68305
(G) TELEFON: 08856/603446
(H) TELEFAX: 08856/603451

(ii) BEZEICHNUNG DER ERFINDUNG: Rekombinante Collagenase Typ II
aus Clostridium histolyticum und ihre Verwendung zur
Isolierung von Zellen und Zellverbaenden

(iii) ANZAHL DER SEQUENZEN: 12

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30B (EPA)

(2) ANGABEN ZU SEQ ID NO: 1:

(i) SEQUENZKENNZEICHEN:
(A) LÄNGE: 487 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 1:

AGATRATTAT GAAAATTTAA CAGTGNCTTT KGTAGCTGAT GATTATTTAG TAAGACATGC 60

TTATAAGAAC CCTAATGAAA TTTATTCTGA AATATCTGAA GTAGCAAAAT TAAAGGATGC 120

TAAGAGTGAA GTTAAGAAAT CACAATATTT TAGTACCTTT ACTTTGAGAG GTAGTTACAC 180

AGGTGGAGTA TCTAAGGGGA AATTAGAAGA TCAAAAAGCA ATGAATAAGT TTATAGATGA 240

TTCACTTAAG AAATTAGATA CGTATTCTTG GAGTGGGTAT AAAACTTTAA CTGCTTATTT 300

CACTAATTAT AAAGTTGACT CTTCAAATAA AGTTACTTAT GATGTAGTAT TCCACGGATA 360

TTTACCAMAT GRAGGTGATT CACGAAATTC ATTACCTTAT GGTAAGACCA ATGGAACTTA 420

TAAGGGGACA GAGAAAGAAA AAATTAAATT TTCTAGTGAA GGCTCTTNCG ATCCAGATGG 480

TAAAATA 487

(2) ANGABEN ZU SEQ ID NO: 2:

(i) SEQUENZKENNZEICHEN:
(A) LÄNGE: 162 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 2:

Asp Xaa Tyr Glu Asn Leu Thr Val Xaa Xaa Val Ala Asp Asp Tyr Leu
1               5                   10                  15

Val Arg His Ala Tyr Lys Asn Pro Asn Glu Ile Tyr Ser Glu Ile Ser
            20                  25                  30

Glu Val Ala Lys Leu Lys Asp Ala Lys Ser Glu Val Lys Lys Ser Gln
        35                  40                  45

Tyr Phe Ser Thr Phe Thr Leu Arg Gly Ser Tyr Thr Gly Gly Val Ser
    50                  55                  60

Lys Gly Lys Leu Glu Asp Gln Lys Ala Met Asn Lys Phe Ile Asp Asp
65                  70                  75                  80

Ser Leu Lys Lys Leu Asp Thr Tyr Ser Trp Ser Gly Tyr Lys Thr Leu
                85                  90                  95

Thr Ala Tyr Phe Thr Asn Tyr Lys Val Asp Ser Ser Asn Lys Val Thr
            100                 105                 110

Tyr Asp Val Val Phe His Gly Tyr Leu Pro Xaa Xaa Gly Asp Ser Arg
        115                 120                 125

Asn Ser Leu Pro Tyr Gly Lys Thr Asn Gly Thr Tyr Lys Gly Thr Glu
    130                 135                 140

Lys Glu Lys Ile Lys Phe Ser Ser Glu Gly Ser Xaa Asp Pro Asp Gly
145                 150                 155                 160

Lys Ile

(2) ANGABEN ZU SEQ ID NO: 3:

(i) SEQUENZKENNZEICHEN:
(A) LÄNGE: 7 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 3:

Tyr Gln Asp His Met Gln Glu
1               5

(2) ANGABEN ZU SEQ ID NO: 4:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: cDNA

(xi) SEQUENZBESCHREIBUNG: SEQ ID NO: 4:

TAYCARGAYC AYATGCARGA R                                    21

(2) ANGABEN ZU SEQ ID NO: 5:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: cDNA

(xi) SEQUENZBESCHREIBUNG: SEQ ID NO: 5:

YTCYTGCATR TGRTCYTGRT A                                    21

(2) ANGABEN ZU SEQ ID NO: 6:

(i) SEQUENZKENNZEICHEN:
(A) LÄNGE: 7 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 6:

Tyr Glx Trp Asp Phe Gly Asp
1               5

(2) ANGABEN ZU SEQ ID NO: 7:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: cDNA

(xi) SEQUENZBESCHREIBUNG: SEQ ID NO: 7:

TAYSARTGGG AYTTYGGNGA Y 21

(2) ANGABEN ZU SEQ ID NO: 8:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: cDNA

(xi) SEQUENZBESCHREIBUNG: SEQ ID NO: 8:

RTCNCCRAAR AAGAAATTA A 21

(2) ANGABEN ZU SEQ ID NO: 9:

(i) SEQUENZKENNZEICHEN:
(A) LÄNGE: 3979 Basenpaare
(B) ART: Nucleotid
(C) STRANGFORM: Doppelstrang
(D) TOPOLOGIE: linear

(ii) ART DES MOLEKÜLS: Genom-DNA

(ix) MERKMAL:
(A) NAME/SCHLÜSSEL: sig-peptide
(B) LAGE:688..846

(ix) MERKMAL:
(A) NAME/SCHLÜSSEL: sig-peptide
(B) LAGE:757..846

(ix) MERKMAL:
(A) NAME/SCHLÜSSEL: CDS
(B) LAGE:847..3792

(xi) SEQUENZBESCHREIBUNG: SEQ ID NO: 9:
ACGAGGGCAG TGCCAAGCTT ATCTTTTGCT ATAAATTTTA AAGAGACACT ATTTATACAG 60
TACCTCATTC CTCCATTTTC AATAGGACCA TCATTAAATA CATGACCTAA ATGAGAGTCA 120
GCATTCTTGC TCCTTACTTC AATTCTGGAC ATACCATGTG AGAAATCTGC TCTTTCTTTT 180
ATATATTTTC TGCTTATGGG TTTTGRAAAA GCAGGCCACC CACATCCAGA ATTAAATTTA 240
TCAATAGATA AGAATAGTGG TTCACCAGAT ACAATGTCAA CATATATACC GTCTTCAAAA 300
TGTTTATCAT ATTTATTTAA AAATGGTTTT TCTGTGGCAT TTCTCTGAGT TACATTATAC 360
TGCAATTCAG TTAAGTTTTC TTTTAATTTC TCGGTATCTA ATTTTYYATA TTCTCTYTCT 420
TTTTTATGGT TCATAAGAAC TCCTCCCGTT TTAAATAGAA TCTTTATAAA TTTATTTTAT 480
CCTAATATTC TCTTATATAC TTAATTAAAT ATTAATAAAA AATTAATGAA CAGGTATATC 540
TTAACAAAAA TTAAACAAAT ATATAACAAA TATTAATAAA TAATGTTGAC ACTACTAAAA 600
AATGGCGTTA TACTTTAATC AAAGGCTTAT ATAATTCCTC AATACAAATA TTCAGATAAT 660
TATGAAAAGG GCATAAGTGA AGGAATTATG AATTTTTTTA AAATTATTTT AAATAGGGGG 720
AAGACTATGA AAAGGAAATG TTTATCTAAA AGGCTTATGT TAGCTATAAC AATGGCTACA 780
ATATTTACAG TGAACAGTAC ATTACCAATT TATGCAGCTG TAGATAAAAA TAATGCAACA 840
GCASCTGTAC AAAATGAAAG TAAGAGGTAT ACAGTATCAT ATTTAAAGAC TTTAAATTAT 900
TATGACTTAG TAGATTTGCT TGCTAAGACT GAAATTGAGA ATTTACCAGA CCTTTTTCAG 960
TATAGCTCAG ATGCAAAAGA GTTCTATGGA AATAAAACTC GTATGAACTT TATTATGGAT 1020
GAAATTGGTA GAAGGGCATC TCAATATACA GAGATAGATC ATAAAGGTAT TCCTACTTTA 1080
GTAGAAGTTG TAAGAGCTGG ATTTTACTTA GGATTCCATA ACAAGGAATT GAATGAAATA 1140
AACAAGAGGT CTTTTAAAGA AAGGGTAATA CCTTCTATAT TAGCAATTCA AAAAAATCCT 1200
AATTTTAAAT TAGGTACTGA AGTTCAAGAT AAAATAGTAT CTGCAACAGG GCTTTTAGCT 1260
GGTAATGAAA CATCCCCTGC AGAAGTTGTA AATAATTTTA CACCAATACT TCAAGACTGT 1320
ATCAAAAATA TGGACAGATA TGCTCTTGAT GATTTAAAGT CAAAAGCATT ATTTAATGTT 1380
TTAGCTGCAC CTACTTATGA TGTGACTGAG TATTTAAGAG CTACTAAAGA AAAACCAGAA 1440
AACACTCCTT GGTATGGTAA AATAGATGGG TTTATAAATG AAGTTAAAAA GTTAGCTCTT 1500
TATGGAAAAA TAAATGATAA GAATTCTTGG ATAATAGATA ACGGTATATA TCATATAGCA 1560
CCTTTAGGGA AGTTACATAG CAATAATAAA ATAGGAATAG AAACTTTAAC AGAGGTTATG 1620

AAAGTTTATC CTTATTTAAG TATGCAACAT TTACAATCAG CAGATCAAAT TAAGCGTCAT 1680

TATGATTCAA AAGATGCTGA AGGAAACAAA ATACCTTTAG ATAAGTTTAA AAAGGAAGGA 1740

AAAGAAAAAT ACTGTCCAAA AACTTATACA TTTGATGATG GAAAAGTAAT AATAAAAGCT 1800

GGTGCTAGAG TAGAAGAAGA AAAAGTTAAA AGACTATACT GGGCATCAAA GGAAGTTAAC 1860

TCTCAATTCT TTAGAGTATA CGGMATAGAC AACCCATTAG AAGAAGGTAA TCCAGATGAT 1920

ATMTTAACAA TGGYTATCTA CAACAGTCCC GAAGAATATA AACTCAATAG TGTTCTATNC 1980

GGATATGATA CTAATAATGG TGGTATGTAT ATAGAGCCAG AAGGMACTTT CTTCACCTAT 2040

GAAAGAGAAG CTCAAGAAAG CACATACACA TTAGRAGAAT TATTTAGACA TGAATATACA 2100

CATTATTTGC AAGGAAGATA TGCAGTTCCA GGACAATGGG GAAGACCCAA ACTTTATGAC 2160

AATGATAGAT TAACTTGGTA TGAAGAAGGT GGAGCAGAAT TATTTGCAGG TTCTACTAGA 2220

ACTTCTGGAA TATTACCAAG AAAGAGTATA GTATCAAATA TTCATAATAC AACAAGAAAT 2280

AATAGATATA AGCTTTCAGA CACTGTACAT TCTAAATATG GTGCTAGTTT TGAATTCTAT 2340

AATTATGCAT GTATGTTTAT GGATTATATG TATAATAAAG ATATGGGTAT ATTAAATAAA 2400

CTAAATGATC TTGCAAAAAA TAATGATGTT GATGGATATG ATAATTATAT TAGAGATTTA 2460

AGTTCTAATC ATGCTTTAAA TGATAAATAT CAAGATCATA TGCAGGAGCG CATAGATAAT 2520

TATGAAAATT TAACAGTGCC TTTTGTAGCT GATGATTATT TAGTAAGACA TGCTTATAAG 2580

AACCCTAATG AAATTTATTC TGAAATATCT GAAGTAGCAA AATTAAAGGA TGCTAAGAGT 2640

GAAGTTAAGA AATCACAATA TTTTAGTACC TTTACTTTGA GAGGTAGTTA CACAGGTGGA 2700

GTATCTAAGG GGAAATTAGA AGATCAAAAA GCAATGAATA AGTTTATAGA TGATTCACTT 2760

AAGAAATTAG ATACGTATTC TTGGAGTGGG TATAAAACTT TAACTGCTTA TTTCACTAAT 2820

TATAAAGTTG ACTCTTCAAA TNAAGTTACT TATGATGTAG TATTCCACGG ATATTTACCA 2880

AATGAAGGTG ATTCAAAAAA TTCATTACCT TATGGTAAGA CCAATGGAAC TTATAAGGGG 2940

ACAGAGAAAG AAAAAATTAA ATTTTCTAGT GAAGGCTCTT TCGATCCAGA TGGTAAAATA 3000

GTTTCTTATG AATGGGATTT CGGAGATGGT AATAAGAGTA ATGAGGAAAA TCCAGAGCAT 3060

TCATATGACA AGGTAGGAAC TTATACAGTA AAATTAAAAG TTACTGATGA CAAGGGAGAA 3120

TCTTCAGTAT CTACTACTAC TGCAGAAATA AGAGATCTTT CAGAAAACAA ACTTCCAGTT 3180

ATATATATGC ATGTACCTAC ATCCGGAGCT TTAAATCAAA AAGTTGTTTT TTATGGAAAA 3240

GGAACATATG ATCCAGATGG ATCTATTGCA GGATATCAAT GGGACTTTGG TGATGGAAGT 3300

GATTTTAGCA GTGAACAAAA CCCAAGCCAC GTATATACTA AAAAAGGTGA ATATACTGTA 3360

ACATTAAGGG TAATGGATAG TAGTGGACAA ATGAGTGAGA AAACTATGAA GATTAAGATT 3420

ACAGATCCGG TATATCCAAT AGGCACTGAA AAAGAACCAA ATAACAGTAA AGAAACCGCA 3480

AGTGGTCCAA TAGTACCAGG TATACCTGTT AGTGGAACCA TAGAAAATAC AAGTGATCAA 3540

GATTATTTCT ATTKTGATGT TATAACACCA GGAGAAGTAA AAATAGATAT AAATAAATTA 3600

GGGTATGGAG GAGCTACTTG GGTAGTATAT GATGAAAATA ATAATGCAGT ATCTTATGCC 3660

ACTGATGATG GACAAAATTT AAGTGGAAAG TTTAAGGCAG ATAAACCAGG TAGATATTAC 3720

ATCCATCTTT ACATGTTTAA TGGTAGTTAT ATGCCATATA GAATTAATAT AGAAGGTTCA 3780

GTAGGAAGAT AATATTTTAT TAGTTGAGGT AACTCCATAT AATAGCTTAG CTATTTCTTA 3840

TGGAGTTACT TTTTATATGT AATAAAATTT TGACTTAAAT TATGAYTTTT TGCTATAATG 3900

GTTTGGAAAT TAATGATTTA TAATTTGAAT TTAATAAAAT TTTGGAGGAA AATTATGATA 3960

CTTYCAGGAA AAGAAATTA 3979

(2) ANGABEN ZU SEQ ID NO: 10:

(i) SEQUENZKENNZEICHEN:
(A) LÄNGE: 981 Aminosäuren
(B) ART: Aminosäure
(C) STRANGFORM:
(D) TOPOLOGIE: linear

(ii) ART DES MOLEKÜLS: Peptid

(xi) SEQUENZBESCHREIBUNG: SEQ ID NO: 10:

Val Gln Asn Glu Ser Lys Arg Tyr Thr Val Ser Tyr Leu Lys Thr Leu
1               5                   10                  15

Asn Tyr Tyr Asp Leu Val Asp Leu Leu Ala Lys Thr Glu Ile Glu Asn
            20                  25                  30

Leu Pro Asp Leu Phe Gln Tyr Ser Ser Asp Ala Lys Glu Phe Tyr Gly
        35                  40                  45

Asn Lys Thr Arg Met Asn Phe Ile Met Asp Glu Ile Gly Arg Arg Ala
    50                  55                  60

Ser Gln Tyr Thr Glu Ile Asp His Lys Gly Ile Pro Thr Leu Val Glu
65                  70                  75                  80

Val Val Arg Ala Gly Phe Tyr Leu Gly Phe His Asn Lys Glu Leu Asn
                85                  90                  95

Glu Ile Asn Lys Arg Ser Phe Lys Glu Arg Val Ile Pro Ser Ile Leu
            100                 105                 110

Ala Ile Gln Lys Asn Pro Asn Phe Lys Leu Gly Thr Glu Val Gln Asp
        115                 120                 125

Lys Ile Val Ser Ala Thr Gly Leu Leu Ala Gly Asn Glu Thr Ser Pro
    130                 135                 140

Ala Glu Val Val Asn Asn Phe Thr Pro Ile Leu Gln Asp Cys Ile Lys
145                 150                 155                 160

Asn Met Asp Arg Tyr Ala Leu Asp Asp Leu Lys Ser Lys Ala Leu Phe
                165                 170                 175

Asn Val Leu Ala Ala Pro Thr Tyr Asp Val Thr Glu Tyr Leu Arg Ala
            180                 185                 190

Thr Lys Glu Lys Pro Glu Asn Thr Pro Trp Tyr Gly Lys Ile Asp Gly
        195                 200                 205

Phe Ile Asn Glu Val Lys Lys Leu Ala Leu Tyr Gly Lys Ile Asn Asp
    210                 215                 220

Lys Asn Ser Trp Ile Ile Asp Asn Gly Ile Tyr His Ile Ala Pro Leu
225                 230                 235                 240

Gly Lys Leu His Ser Asn Asn Lys Ile Gly Ile Glu Thr Leu Thr Glu
                245                 250                 255

Val Met Lys Val Tyr Pro Tyr Leu Ser Met Gln His Leu Gln Ser Ala
            260                 265                 270

Asp Gln Ile Lys Arg His Tyr Asp Ser Lys Asp Ala Glu Gly Asn Lys
        275                 280                 285

Ile Pro Leu Asp Lys Phe Lys Lys Glu Gly Lys Glu Lys Tyr Cys Pro
    290                 295                 300

Lys Thr Tyr Thr Phe Asp Asp Gly Lys Val Ile Ile Lys Ala Gly Ala
305                 310                 315                 320

Arg Val Glu Glu Glu Lys Val Lys Arg Leu Tyr Trp Ala Ser Lys Glu
                325                 330                 335

Val Asn Ser Gln Phe Phe Arg Val Tyr Gly Ile Asp Asn Pro Leu Glu
            340                 345                 350

Glu Gly Asn Pro Asp Asp Ile Leu Thr Met Xaa Ile Tyr Asn Ser Pro
        355                 360                 365

Glu Glu Tyr Lys Leu Asn Ser Val Leu Xaa Gly Tyr Asp Thr Asn Asn
    370                 375                 380

Gly Gly Met Tyr Ile Glu Pro Glu Gly Thr Phe Phe Thr Tyr Glu Arg
385                 390                 395                 400

Glu Ala Gln Glu Ser Thr Tyr Thr Leu Xaa Glu Leu Phe Arg His Glu
                405                 410                 415

Tyr Thr His Tyr Leu Gln Gly Arg Tyr Ala Val Pro Gly Gln Trp Gly
            420                 425                 430

Arg Pro Lys Leu Tyr Asp Asn Asp Arg Leu Thr Trp Tyr Glu Glu Gly
        435                 440                 445

Gly Ala Glu Leu Phe Ala Gly Ser Thr Arg Thr Ser Gly Ile Leu Pro
    450                 455                 460

Arg Lys Ser Ile Val Ser Asn Ile His Asn Thr Thr Arg Asn Asn Arg
465                 470                 475                 480

Tyr Lys Leu Ser Asp Thr Val His Ser Lys Tyr Gly Ala Ser Phe Glu
                485                 490                 495

Phe Tyr Asn Tyr Ala Cys Met Phe Met Asp Tyr Met Tyr Asn Lys Asp
            500                 505                 510

Met Gly Ile Leu Asn Lys Leu Asn Asp Leu Ala Lys Asn Asn Asp Val
        515                 520                 525

Asp Gly Tyr Asp Asn Tyr Ile Arg Asp Leu Ser Ser Asn His Ala Leu
    530                 535                 540

Asn Asp Lys Tyr Gln Asp His Met Gln Glu Arg Ile Asp Asn Tyr Glu
545                 550                 555                 560

Asn Leu Thr Val Pro Phe Val Ala Asp Asp Tyr Leu Val Arg His Ala
                565                 570                 575

Tyr Lys Asn Pro Asn Glu Ile Tyr Ser Glu Ile Ser Glu Val Ala Lys
            580                 585                 590

Leu Lys Asp Ala Lys Ser Glu Val Lys Lys Ser Gln Tyr Phe Ser Thr
        595                 600                 605

Phe Thr Leu Arg Gly Ser Tyr Thr Gly Gly Val Ser Lys Gly Lys Leu
    610                 615                 620

Glu Asp Gln Lys Ala Met Asn Lys Phe Ile Asp Asp Ser Leu Lys Lys
625                 630                 635                 640

Leu Asp Thr Tyr Ser Trp Ser Gly Tyr Lys Thr Leu Thr Ala Tyr Phe
                645                 650                 655

Thr Asn Tyr Lys Val Asp Ser Ser Asn Xaa Val Thr Tyr Asp Val Val
            660                 665                 670

Phe His Gly Tyr Leu Pro Asn Glu Gly Asp Ser Lys Asn Ser Leu Pro
        675                 680                 685

Tyr Gly Lys Thr Asn Gly Thr Tyr Lys Gly Thr Glu Lys Glu Lys Ile
    690                 695                 700

Lys Phe Ser Ser Glu Gly Ser Phe Asp Pro Asp Gly Lys Ile Val Ser
705                 710                 715                 720

Tyr Glu Trp Asp Phe Gly Asp Gly Asn Lys Ser Asn Glu Glu Asn Pro
                725                 730                 735

Glu His Ser Tyr Asp Lys Val Gly Thr Tyr Thr Val Lys Leu Lys Val
            740                 745                 750

Thr Asp Asp Lys Gly Glu Ser Ser Val Ser Thr Thr Thr Ala Glu Ile
        755                 760                 765

Arg Asp Leu Ser Glu Asn Lys Leu Pro Val Ile Tyr Met His Val Pro
    770                 775                 780

Thr Ser Gly Ala Leu Asn Gln Lys Val Val Phe Tyr Gly Lys Gly Thr
785                 790                 795                 800

Tyr Asp Pro Asp Gly Ser Ile Ala Gly Tyr Gln Trp Asp Phe Gly Asp
                805                 810                 815

Gly Ser Asp Phe Ser Ser Glu Gln Asn Pro Ser His Val Tyr Thr Lys
            820                 825                 830

Lys Gly Glu Tyr Thr Val Thr Leu Arg Val Met Asp Ser Ser Gly Gln
        835                 840                 845

Met Ser Glu Lys Thr Met Lys Ile Lys Ile Thr Asp Pro Val Tyr Pro
    850                 855                 860

Ile Gly Thr Glu Lys Glu Pro Asn Asn Ser Lys Glu Thr Ala Ser Gly
865                 870                 875                 880

Pro Ile Val Pro Gly Ile Pro Val Ser Gly Thr Ile Glu Asn Thr Ser
                885                 890                 895

Asp Gln Asp Tyr Phe Tyr Xaa Asp Val Ile Thr Pro Gly Glu Val Lys
            900                 905                 910

Ile Asp Ile Asn Lys Leu Gly Tyr Gly Gly Ala Thr Trp Val Val Tyr
        915                 920                 925

Asp Glu Asn Asn Asn Ala Val Ser Tyr Ala Thr Asp Asp Gly Gln Asn
    930                 935                 940

Leu Ser Gly Lys Phe Lys Ala Asp Lys Pro Gly Arg Tyr Tyr Ile His
945                 950                 955                 960

Leu Tyr Met Phe Asn Gly Ser Tyr Met Pro Tyr Arg Ile Asn Ile Glu
                965                 970                 975

Gly Ser Val Gly Arg
            980

(2) ANGABEN ZU SEQ ID NO: 11:

(i) SEQUENZKENNZEICHEN:
(A) LÄNGE: 41 Basenpaare
(B) ART: Nucleotid
(C) STRANGFORM: Einzelstrang
(D) TOPOLOGIE: linear

(ii) ART DES MOLEKÜLS: cDNA

(xi) SEQUENZBESCHREIBUNG: SEQ ID NO: 11:

GGATCCCGGG TTATGAATTT TTTTAAAATT ATTTTAAATA G 41

(2) ANGABEN ZU SEQ ID NO: 12:

(i) SEQUENZKENNZEICHEN:
(A) LÄNGE: 40 Basenpaare
(B) ART: Nucleotid
(C) STRANGFORM: Einzelstrang
(D) TOPOLOGIE: linear

(ii) ART DES MOLEKÜLS: cDNA

(xi) SEQUENZBESCHREIBUNG: SEQ ID NO: 12:

CTGCATGCGG CCGCTTATTA TCTTCCTACT GAACCTTCTA 40

## Beschreibung

Gegenstand der Erfindung ist eine rekombinante Collagenase Typ II aus Clostridium histolyticum und ihre Verwendung zur Isolierung von Zellen und Zellverbänden.

Bakterielle Collagenasen, z.B. aus Clostridium histolyticum, werden zum Verdau von Geweben und zur Gewinnung von Einzelzellen oder Zellverbänden (z.B. Inseln) verwendet (Inseln: Sutton et al., Transplantation 42 (1986) 689 - 691; Leber: Quibel et al., Anal. Biochem. 154 (1986) 26 - 28; Knochen: Hefley et al., J. Bone Mineral Res. 2 (1987) 505 - 516; Nabelschnur: Holzinger et al., Immunol. Lett. 35 (1993) 109 - 118. Aus Clostridium histolyticum sind zwei verschiedene Collagenase-Typen bekannt (M.F. French et al., J. Protein Chemistry 11 (1992) 83 - 97). Die Herstellung von Collagenase Class II aus Clostridium histolyticum (CHC II) ist beispielsweise beschrieben in E.L. Angleton und H.E. van Wart, Biochemistry 27 (1988) 7413 - 7418 und 7406 - 7412. Es hat sich jedoch gezeigt, daß CHC II sehr empfindlich auf wiederholtes Einfrieren und Auftauen ist. Nach 12 Einfrier-/Auftauzyklen ist die Aktivität von CHC II praktisch vollständig verschwunden (T.J. Hefley, J. Bone and Mineral Res. 2 (1987) 505-516).

Aufgabe der vorliegenden Erfindung ist die Bereitstellung einer gefrierstabilen Collagenase Class II aus Clostridium histolyticum.

Die Aufgabe wird gelöst durch ein Polypeptid, welches die Eigenschaften einer Collagenase Class II aus Clostridium histolyticum hat, nach 12 Einfrier-/Auftauzyklen noch in beträchtlichem Umfang Collagenase-Aktivität besitzt und erhältlich ist durch Expression einer exogenen DNA-Sequenz in prokaryontischen oder eukaryontischen Wirtszellen und Isolierung des gewünschten Polypeptids, wobei die DNA-Sequenz im für diese Collagenase charakteristischen Bereich SEQ ID NO:1 oder eine Sequenz, welche im Rahmen der Degeneration des genetischen Codes für die gleiche Aminosäuresequenz codiert, enthält.

Es hat sich überraschenderweise gezeigt, daß eine CHC II, welche das Produkt einer rekombinanten Herstellung ist, gegenüber wiederholtem Einfrieren und Auftauen wesentlich stabiler als die aus natürlichen Quellen isolierte CHC II ist und nach 12 Einfrier-/Auftauzyklen noch in beträchtlichem Umfang Collagenase-Aktivität besitzt (vorzugsweise mehr als 50%, besonders bevorzugt mehr als 80% der Ausgangsaktivität).

Die Herstellung der rekombinanten CHC II kann nach den dem Fachmann geläufigen Methoden erfolgen.

Dazu wird zunächst eine DNA-Sequenz hergestellt, welche in der Lage ist, ein Protein zu produzieren, welches die Aktivität von CHC II besitzt. Unter einer solchen DNA-Sequenz im Sinne der Erfindung ist ein isoliertes DNA-Molekül zu verstehen. Die das Protein codierende Sequenz kann mit Hilfe von SEQ ID NO:1 ermittelt werden, indem man den Leserahmen anhand eines Vergleichs mit bekannten Peptidsequenzen von Collagenasen festlegt und die so ermittelte DNA-Sequenz mit Hilfe des genetischen Codes in eine Aminosäuresequenz umsetzt. Die das aktive Protein codierende Sequenz ist ca. 2 bis 3 mal länger als SEQ ID NO:1. Die DNA-Sequenz wird in einen Vektor kloniert, der in eine Wirtszelle transferiert werden kann und dort replizierbar ist. Ein derartiger Vektor enthält zusätzlich zur CHC II-Sequenz Operator-Elemente, die zur Expression der DNA-Sequenz erforderlich sind. Dieser Vektor, der die CHC II-Sequenz und die Operator-Elemente enthält, wird in einen Vektor transferiert, der in der Lage ist, die DNA von CHC II zu exprimieren. Die Wirtszelle wird unter Bedingungen, die zur Amplifikation des Vektors geeignet sind, kultiviert und CHC II gewonnen. Dabei wird durch geeignete Maßnahmen sichergestellt, daß das Protein eine aktive tertiäre Struktur einnehmen kann, in der es CHC II-Eigenschaften zeigt.

Die Klonierung und die Nukleotidsequenz von CHC II ist in der nachveröffentlichten Publikation K. Yoshihara et al., J. Bacteriol. 176 (Nov. 1994) 6489 - 6494 beschrieben.

Dabei ist es nicht erforderlich, daß das exprimierte Protein die exakte CHC II-Aminosäuresequenz mit dem Teilbereich SEQ ID NO: 2 enthält. Ebenso geeignet sind Proteine, welche im wesentlichen die gleiche Sequenz enthalten und analoge Eigenschaften zeigen.

Auch die Nukleinsäuresequenz des Proteins kann modifiziert sein. Derartige Modifikationen sind beispielsweise:
- Veränderung der Nukleinsäure, um verschiedene Erkennungssequenzen von Restriktionsenzymen zur Erleichterung der Schritte der Ligation, Klonierung und Mutagenese einzuführen
- Veränderung der Nukleinsäure zum Einbau von bevorzugten Codons für die Wirtszelle
- Ergänzung der Nukleinsäure um zusätzliche Operator-Elemente, um die Expression in der Wirtszelle zu optimieren.

Ein weiterer Gegenstand der Erfindung ist ein Verfahren zur Herstellung eines Polypeptids, welches die Eigenschaften einer Collagenase Class II aus Clostridium histolyticum hat und nach 12 Einfrier-/Auftauzyklen noch in beträchtlichem Umfang Collagenase-Aktivität besitzt, durch Expression einer exogenen DNA-Sequenz in prokaryontischen oder eukaryontischen Wirtszellen und Isolierung des gewünschten Polypeptids, wobei die DNA-Sequenz im für diese Collagenase charakteristischen Bereich SEQ ID NO:1 oder eine Sequenz, welche im Rahmen der Degeneration des genetischen Codes für die gleiche Aminosäuresequenz codiert, enthält.

Die Expression des Proteins erfolgt vorzugsweise in Mikroorganismen, insbesondere in Prokaryonten, und dort in E. coli.

Die Expressionsvektoren müssen einen Promotor enthalten, der die Expression des Proteins im Wirtsorganismus erlaubt. Derartige Promotoren sind dem Fachmann bekannt und sind beispielsweise lac Promotor (Chang et al., Nature 198 (1977) 1056), trp Promotor (Goeddel et al., Nuc. Acids Res. 8 (1980) 4057), λ_{PL} Promotor (Shimatake et al., Nature 292 (1981) 128) und T5 Promotor (US-Patent Nr. 4,689,406). Ebenfalls geeignet sind synthetische Promotoren wie beispielsweise tac Promotor (US-Patent Nr. 4,551,433). Ebenso geeignet sind gekoppelte Promotorsysteme wie beispielsweise T7-RNA-Polymerase/Promotorsystem (Studier et al., J. Mol. Biol. 189 (1986) 113). Ebenso geeignet sind hybride Promotoren aus einem Bacteriophagen-Promotor und der Operator-Region des Mikroorganismus (EP-A 0 267 851). Zusätzlich zum Promotor ist eine effektive Ribosomenbindungsstelle erforderlich. Für E. coli wird diese Ribosomenbindungsstelle als Shine-Dalgarno (SD)-Sequenz bezeichnet (Sambrook et al., "Expression of cloned genes in E. coli" in Molecular Cloning: A laboratory manual (1989) Cold Spring Harbor Laboratory Press, New York, USA).

Zur Verbesserung der Expression ist es möglich, das Protein als Fusionsprotein zu exprimieren. In diesem Fall wird üblicherweise eine DNA-Sequenz, welche für den N-terminalen Teil eines endogenen bakteriellen Proteins oder ein anderes stabiles Protein codiert, an das 5'-Ende der für CHC II codierenden Sequenz fusioniert. Beispiele hierfür sind beispielsweise lacZ (Phillips and Silhavy, Nature 344 (1990) 882 - 884), trpE (Yansura , Meth. Enzymol. 185 (1990) 161-166).

Nach Expression des Vektors, vorzugsweise eines biologisch funktionellen Plasmids oder eines viralen Vektors, werden die Fusionsproteine vorzugsweise mit Enzymen (z.B. Faktor Xa) gespalten (Nagai et al., Nature 309 (1984) 810). Weitere Beispiele für die Spaltstellen die IgA-Protease-Spaltstelle (WO 91/11520, EP-A 0 495 398) und die Ubiquitin-Spaltstelle (Miller et al., Bio/Technology 7 (1989) 698).

Die auf diese Weise in Bakterien exprimierten Proteine werden durch Aufschluß der Bakterien und Proteinisolierung in üblicher Weise gewonnen.

In einer weiteren Ausführungsform ist es möglich, die Proteine als aktive Proteine aus den Mikroorganismen zu sezernieren. Hierzu wird vorzugsweise ein Fusionsprodukt verwendet, welches aus der Signalsequenz, die für die Sekretion von Proteinen in den verwendeten Wirtsorganismen geeignet ist, und der Nukleinsäure, welche für das Protein codiert, besteht. Das Protein wird dabei entweder in das Medium (bei grampositiven Bakterien) oder in den periplasmatischen Raum (bei gramnegativen Bakterien) sezerniert. Zwischen der Signalsequenz und der für CHC II codierenden Sequenz ist zweckmäßig eine Spaltstelle angebracht, die entweder bei der Prozessierung oder in einem zusätzlichen Schritt die Abspaltung des Proteins erlaubt. Derartige Signalsequenzen stammen beispielsweise von ompA (Ghrayeb et al., EMBO J. 3 (1984) 2437), phoA (Oka et al., Proc. Natl. Acad. Sci. USA 82 (1985) 7212).

Zusätzlich enthalten die Vektoren noch Terminatoren. Terminatoren sind DNA-Sequenzen, die das Ende eines Transkriptionsvorganges signalisieren. Sie zeichnen sich meist durch zwei strukturelle Eigenarten aus: eine umgekehrt repetitive G/C-reiche Region, die intramolekular eine Doppelhelix bilden kann, sowie eine Anzahl von U(bzw. T)-Resten. Beispiele sind der Haupt-Terminator in der DNA des Phagen fd (Beck und Zink, Gene 16 (1981) 35-58) sowie rrnB (Brosius et al., J. Mol. Biol. 148 (1981) 107 - 127).

Zusätzlich enthalten die Expressionsvektoren üblicherweise einen selektierbaren Marker, um transformierte Zellen zu selektieren. Derartige selektierbare Marker sind beispielsweise die Resistenzgene für Ampicillin, Chloramphenicol, Erythromycin, Kanamycin, Neomycin und Tetracyclin (Davies et al., Ann. Rev. Microbiol. 32 (1978) 469). Ebenso geeignete selektierbare Marker sind die Gene für essentielle Substanzen der Biosynthese von für die Zelle notwendigen Stoffen wie z.B. Histidin, Tryptophan und Leucin.

Es sind eine Vielzahl von geeigneten bakteriellen Vektoren bekannt. Beispielsweise sind für die folgenden Bakterien Vektoren beschrieben: Bacillus subtilis (Palva et al., Proc. Natl. Acad. Sci. USA 79 (1982) 5582), E. coli (Aman et al., Gene 40 (1985) 183; Studier et al., J. Mol. Biol. 189 (1986) 113), Streptococcus cremoris (Powell et al., Appl. Environ. Microbiol. 54 (1988) 655), Streptococcus lividans und Streptomyces lividans (US-Patent Nr. 4,747,056).

Weitere gentechnologische Verfahren zur Herstellung und Expression von geeigneten Vektoren sind in J. Sambrook et al., Molecular Cloning: a laboratory manual (1989), Cold Spring Harbor Laboratory Press, New York, N.Y. beschrieben.

Eine Expression von rekombinanter CHC II ist außer in prokaryontischen Mikroorganismen auch in Eukaryonten (wie beispielsweise CHO-Zellen, Hefe oder Insektenzellen) möglich. Als eukaryontisches Expressionssystem wird das Hefesystem oder Insektenzellen bevorzugt. Die Expression in Hefe kann über drei Arten von Hefevektoren (integrierende YI_{P} (yeast integrating plasmids)-Vektoren, replizierende YR_{P} (yeast replicon plasmids)-Vektoren und episomale YE_{P} (yeast episomal plasmids)-Vektoren erfolgen. Näheres hierzu ist beispielsweise in S.M. Kingsman et al, Tibtech 5 (1987) 53-57 beschrieben.

Ein weiterer Gegenstand der Erfindung ist ein Verfahren zur Auflösung von Zellgewebe und Freisetzung von darin enthaltenen Zellen oder Zellverbänden durch Inkubation des Zellgewebes mit einer Collagenase Class II aus Clostridium histolyticum, welche nach 12 Einfrier/Auftauzyklen noch in beträchtlichem Umfang Collagenase-Aktivität besitzt und in einem charakteristischen Teilbereich SEQ ID NO:2 enthält oder in diesem Bereich von SEQ ID NO: 1 oder einer Sequenz, welche im Rahmen der Degeneration des genetischen Codes für die gleiche Aminosäuresequenz codiert wird und das Produkt einer prokaryontischen oder eukaryontischen Expression einer exogenen DNA ist, bis zur Freisetzung der Zellen oder Zellverbände im gewünschten Umfang und Abtrennung der Zellen oder der Zellverbände von den Zellgewebeanteilen. Die Abtrennung der Zellen oder Zellverbände von den Zellgewebsanteilen erfolgt bevorzugt durch Zentrifugation mit einem Dichtegradienten.

Eine Isolierung von Zellen bzw. Zellverbänden aus Geweben (z.B. Pankreas, Leber, Haut, Endothel, Nabelschnur, Knochen, Tumorgewebe) erfolgt im allgemeinen durch Inkubation von Organen, Organteilen oder Geweben mit Enzymen, die die umgebende extrazelluläre Bindegewebe-Matrix auflösen (Inseln: Sutton et al., Transplantation 42 (1986) 689 - 691; Leber: Quibel et al., Anal. Biochem. 154 (1986) 26 - 28; Knochen: Hefley et al., J. Bone Mineral Res. 2 (1987) 505 - 516; Nabelschnur: Holzinger et al., Immunol. Lett. 35 (1993) 109 - 118. Derartig isolierte Tumorzellen können vorteilhaft zur Tumorvakzinierung und/oder adoptiven Immuntherapie verwendet werden. Eine Gewebsintegration kann auch durch Perfusion des gesamten Organs (Ricordi et al, Diabetes 37 (1988) 413 - 420) mit Enzymlösung erfolgen. Entscheidend neben der Zusammensetzung des Enzymgemisches ist dabei die Dauer, der pH-Wert und die Temperatur des Verdaus sowie auch die mechanische Einwirkung, z.B. durch Schütteln und Zusatz von Metallkugeln. Da extrazelluläre Bindegewebsmatrix oft einen hohen Collagen-Anteil aufweist, kommt den Collagenasen, und hier speziell der Collagenase Typ II, eine besondere Rolle zu. (Wolters, Hormone and Metabolic Research 26 (1994) p. 80)

Vorzugsweise wird das erfindungsgemäße Verfahren zur Isolierung von Inseln oder Inselzellen aus Pankreasgewebe verwendet.

Daneben kann der Zusatz weiterer Enzyme, wie Proteinasen (z. B. neutrale Protease, vgl. Bsp. 5, oder andere Metalloproteasen; Serinproteasen, wie Trypsin, Chymotrypsin, Plasmin etc.; Cysteinproteasen; Aspartat-Proteasen), Elastasen, Hyaluronidasen, Lipasen oder anderer Collagenasen für die Qualität des Verdaus von Vorteil sein.

Die Erfindung wird durch folgende Beispiele und Sequenzprotokolle näher erläutert.

In den Sequenzprotokollen bedeuten:
SEQ ID NO: 1: DNA-Fragment von CHC II
SEQ ID NO: 2: abgeleitetes Proteinfragment
SEQ ID NO: 3 - 8: Primersequenzen
SEQ ID NO: 9: cDNA von CHC II
SEQ ID NO: 10: Proteinsequenz von CHC II
SEQ ID NO: 11/12: Primer

### Beispiel 1

### Reinigung der Collagenase Class II (CHC II)

1 g Collagenase P (aus Clostridium histolyticum, Boehringer Mannheim GmbH, Best.-Nr. 1213857) wurde in 20 ml H₂O (Milli Q-Qualität) gelöst und partikuläre Bestandteile durch Zentrifugation abgetrennt. Nach 60% Ammoniumsulfat-Fällung wurde der Niederschlag in 9.5 ml H₂O aufgenommen und über Nacht bei 4°C gegen 10 mM Tris-HCl, pH 7,5 dialysiert.

Das Dialysat wurde bei Raumtemperatur auf eine Zn-Chelat-Sepharose-Säule (Säulenvolumen SV = 50 ml, 2 cm Durchmesser) mit einem Fluß von 1,5 SV (75 ml/h ) aufgetragen. Danach wurde die Säule mit 10 mM Tris-HCl, pH 7,5 gewaschen, bis die Grundlinie im UV-Chromatogramm wieder erreicht war. Die Elution der an die Affinitätsmatrix gebundenen CHC II erfolgte mit einem linearen Gradienten (10 SV = 500 ml) bis 50 mM Tris, pH 7,5, 50 mM Imidazol. Die aktiven Fraktionen (Bestimmung durch Spaltung synthetischer Peptidsubstrate) wurden gegen 10 mM Tris, pH 8,0, 1 mM CaCl₂ dialysiert und bei -20°C gelagert.

Im SDS-Gel tritt eine starke Anreicherung der Banden im Bereich > 85 kD hervor, weitere Proteine (MW zwischen 40 und 85 kD) sowie wenige kleinere Banden sind erkennbar.

Die CHC II-enthaltenden Fraktionen wurden über eine MonoQ-Säule (FPLC) in 10 mM Tris-HCl, pH 8.0 unter Verwendung eines linearen NaCl-Gradienten in 30 min von 0 auf 0.3 M NaCl in 10 mM Tris-HCl, pH 8.0 weiter aufgetrennt. Die CHC II-Aktivität enthaltenden Peaks werden über Reversed-Phase-HPLC weiter gereinigt (Laufmittel A: 0,12% TFA / H₂O; Laufmittel B: 0,12% TFA (Trifluoressigsäure) / 100% Acetonitril). Insgesamt werden 4 Peaks gesammelt, zur Trockene eingeengt und nach Carboxymethylierung einem LysC-Verdau unterworfen. Die Trennung der Peptide erfolgte ebenfalls über Reversed-Phase-HPLC an einer C₄ Säule mit einem Gradienten von 5 - 45% Acetonitril / 0.12% TFA. Die Chromatogramme aller Verdaus waren bis auf minimale Unterschiede identisch. Sie unterschieden sich deutlich von den LysC-Peptide-Maps der Collagenase I (gereinigt über Gelfiltration an Superdex HiLoad S 200 bzw. Nachreinigung des Durchlaufs der Zn-Chelat-Sepharose). Die Peptide wurden gesammelt, zur Trockene eingeengt und sequenziert.

### Beispiel 2

### Klonierung der Collagenase II aus Clostridium histolyticum

Von nach der Aufreinigung der CHC II gemäß Beispiel 1 ermittelten Peptidsequenzen ausgehend wird die zugehörige (degenerierte) DNA-Sequenz abgeleitet. Sequenzen, die eine vorteilhafte (geringe) Denaturierung zeigen, werden verwendet, um z.B. via PCR eine markierte DNA Probe zum Screenen von Genbanken herzustellen.

Besonders geeignet sind z.B. 2 Peptide Coll-2-60 und Coll-2-51, von denen sich folgende Primer ableiten lassen:
Peptid Coll-2-60 (SEQ ID NO:3)
Primer Coll-2-60F (SEQ ID NO:4)
Primer Coll-2-60R (SEQ ID NO:5)
Peptid Coll-2-51 (SEQ ID NO:6)
Primer Coll-2-51F (SEQ ID NO:7)
Primer Coll-2-51R (SEQ ID NO:8)
Mit Primer 60F und 51R erhält man nach PCR mit nach herkömmlichen Methoden isolierter DNA aus Clostridium histolyticum Fragmente von ca. 500 und ca. 750 bp Länge.

Das 500 bp Fragment wird mit dig-dUTP (hergestellt nach WO 89/06698, EP-A 0 324 468) markiert, z.B. ebenfalls in einer PCR Reaktion, und dient zur Identifizierung positiver Klone aus einer Genbank. Die Genbank kann in allgemein bekannter Weise aus Clostridium histolyticum DNA nach Verdau mit Restriktionsenzymen hergestellt werden.

Das 500 bp Fragment wurde sequenziert und enthält DNA der Sequenz SEQ ID NO:1.

Das 750 bp Fragment kann mit PstI in ein ca. 600 bp großes und ein 150 bp großes Fragment zerlegt werden. Das 600 bp Fragment enthält ebenfalls SEQ ID NO:1. Ein Vektor, der das 600 bp Fragment enthält ist pC2-600 und wurde unter DSM 8996 am 24.02.1994 bei der DSM (Deutsche Sammlung von Mikroorganismen und Zellkulturen GmbH, Mascheroder Weg 1b, D-38124 Braunschweig) hinterlegt.

Die Aminosäuresequenz zeigt zu ca. 25 % Homologie zu einer Collagenase aus Vibrio alginolyticus und ist in SEQ ID NO:2 dargestellt. SEQ ID NO:2 wird von den Nukleotiden 2 - 487 aus SEQ ID NO:1 codiert.

Durch inverse PCR können nach Verdau der genomischen DNA von Clostridium histolyticum mit geeigneten Restriktionsenzymen mit aus SEQ ID NO: 1 abgeleiteten Primern die fehlenden Teile der codierenden Region und der untranslatierten Bereiche des Gens amplifiziert und sequenziert werden. Die codierende Region des Gens (ca. 3 kB) ist in SEQ ID NO: 1 enthalten.

Der Leserahmen von SEQ ID NO: 9 kann mit Hilfe von durch Proteinsequenzierung gefundenen Peptidsequenzen eindeutig festgelegt werden und die Sequenz kann in die Peptidsequenz SEQ ID NO: 10 übersetzt werden.

Die Enden des codierenden Bereichs von Nukleotid 688 bis Nukleotid 3792 wurden mit Hilfe zweier Primer (SEQ ID NO: 11 und 12) modifiziert und via XmaI und NotI in pUCBM 20 kloniert. Das resultierende Plasmid pC2-Ex1/3-23 wurde unter DSM 9579 bei der Deutschen Sammlung von Mikroorganismen und Zellkulturen GmbH, Mascheroder Weg 1b, D-38124 Braunschweig, am 09.12.1994 hinterlegt.

### Beispiel 3

### Expression von Collagenase Class II

DNA-Fragmente, die das Gen für CHC II oder Teile davon enthalten, werden sequenziert und in geeigneter Weise an den Enden modifiziert (z.B. via PCR) und als Ganzes oder in Kombination in einen Expressionsvektor für E. coli eingesetzt. Als Promotor wird der tac Promotor verwendet. Die Verwendung anderer, vorzugsweise gut regulierbarer Promotoren ist jedoch auch möglich.

Zur Expression kann ein Fragment von SEQ ID NO: 9 verwendet werden, das an Position 688 oder 757 beginnt und im Bereich von Position 3792 - Position 390° endet, unter der Kontrolle eines der üblichen Promotoren gebracht werden. Alternativ dazu kann ein Fragment, das an Position 847 beginnt und im Bereich von Position 3792 - Position 3900 endet, zuvor noch mit der Sequenz für ein heterologes Signalpeptid versehen werden und dann hinter die üblichen Promotoren gesetzt werden. Als Vektoren sind solche mit hoher Kopienzahl, wie die der pUC-Serie geeignet. Ebenso können aber auch Vektoren mit niedrigerer Kopienzahl abgeleitet von pACYC 177oder pACYC 184 benutzt werden, wenn die gewählte Wirtszelle die Expression in hoher Kopienzahl nicht toleriert. Als Wachstumstemperatur ist ein Bereich von 25 - 37 ⁰C bevorzugt.

Ein E. coli Stamm, der mit einem Expressionsplasmid transformiert worden ist, wird entweder in Minimalmedium oder LB Medium unter Antibiotika-Selektion über Nacht bei 30 oder 37 °C angezogen (z.B. in 5 ml Kultur). Die Übernachtkultur wird in ein größeres Volumen (z.B. 1 l) überimpft und weiterwachsen lassen. Verwendet man dieses Volumen bereits zur Biomassengewinnung kann man bei einer OD⁵⁵⁰ von 0.2 bis 2 mit IPTG induzieren und die Zellen weiterwachsen lassen, bis die OD und/oder die gebildete Enzymaktivität nicht weiter zunimmt. Zu diesem Zeitpunkt wird abzentrifugiert und die Biomasse zur Aufreinigung von Collagenase verwendet.

Falls sich ein erheblicher Teil der Collagenase-Aktivität im Medium befindet; wird auch das Medium gesammelt und Collagenase daraus aufgereinigt.

### Beispiel 4

### Inselisolierung aus Schweine-Pankreas

Aus einem frisch geschlachteten Schwein wird der Pankreas präpariert und in eiskaltem HBSS-Puffer (Gibco) bis zur weiteren Verarbeitung gekühlt. In den Ductus pancreaticus wird eine Braunüle eingeführt und befestigt, die Dichtigkeit des Pankreas wird mit HBSS-Puffer geprüft. Eine Enzymlösung in HBSS-Puffer + Ca²⁺, die die gereinigte rekombinante Collagenase Typ II allein oder in Verbindung mit einer gereinigten nativen oder rekombinanten neutralen Protease aus *Clostridium histolyticum* enthält, wird eingespritzt. Der so behandelte Pankreas wird an die ebenfalls obige Enzymlösung enthaltende Perfusionseinheit (diskontinuierliche Perfusion) angeschlossen. Der Verdau erfolgt zwischen 4°C - 37°C in einem Zeitraum von 5 bis 120 Minuten. Nach der als optimal angenommenen Zeit wird die Pumpe gestoppt und das den Pankreas enthaltende Gefaß 3 bis 20 Minuten lang vorsichtig per Hand geschüttelt. Im Bedarfsfall vorher zugegebene Metallkugeln erleichtern zusätzlich die mechanische Dissoziation des Gewebes und die Freisetzung der Inseln aus dem umgebenden exokrinen Gewebe.

Der Verdau wird durch Zugabe von eiskaltem HBSS/10 % FKS (fötales Kälberserum) gestoppt und die Suspension durch ein Sieb (Maschengröße 300 µm) filtriert, um die Grobteile abzutrennen. Die im Durchfluß befindlichen Inseln werden 10 Minuten bei 100 g in 250 ml Nalgene-Rundbodenflaschen abzentrifugiert. Der Überstand wird abgenommen und das die Inseln enthaltende Pellet in 50 ml FKS resuspendiert.

Eine weitere Aufreinigung der Inseln kann über einen per Hand hergestellten Dichtegradienten erfolgen. In 250 ml Nalgene-Rundbodenflaschen werden zunächst 7 ml der Inselsuspension gegeben. Diese wird zunächst mit 93 ml einer Ficoll-Lösung® (φ = 1.077 g/cm³), darauffolgend mit 50 ml Medium (RPMI 1640) überschichtet. Diese Gradienten werden im Ausschwingrotor bei 100 g für 10 Minuten zentrifugiert. Fraktionen á 10 ml werden abgenommen, die Größe, Reinheit und Ausbeute der mit Dithizon gefärbten Inseln wird in jeder Fraktion mikroskopisch oder mit Hilfe der Bildanalytik bestimmt.

### Beispiel 5

### Isolierung von neutraler Protease

Collagenase P wird in 5 mM HEPES-Puffer, pH 7.5, 1 mM CaCl₂ gelöst (c = 50 mg/ml und abzentrifugiert. Ein Aliquot (10 mg) wird auf eine MonoQ-Säule (Pharmacia) aufgespritzt (FPLC) und mit einem CaCl₂-Gradienten (bis 150 mM in 20 min) eluiert. Die Fraktion mit der höchsten spezifischen casinolytischen Aktivität (bestimmt über Resorufin-markiertes Casein, Boehringer Mannheim GmbH) enthielt ein Protein mit einem MW von 30 - 35 kDa (SDS-Gel). Nach Carboxymethylierung und LysC-Verdau wurden die entstandenen Peptide über Reversed-Phase HPLC an einer C₈-Säule aufgetrennt, zur Trockene eingeengt und sequenziert. Alternativ dazu wurden die Proteinfraktionen mit hoher caseinolytischer Aktivität über ein SDS-Gel aufgetrennt, auf PVDF-Membranen (Millipore) transferiert (Western-Blot; Transferpuffer 50 mM Tris, 50 mM Borsäure, pH 8.3) und nach Amidoschwarz-Färbung die entsprechende Bande ausgeschnitten. Nach LysC-Verdau des immobilisierten Proteins wurden die Peptide mit HCOOH abgelöst und, wie oben beschrieben, getrennt und sequenziert.

## Patentansprüche

1. Verfahren zur Auflösung von Zellgewebe und Freisetzung von darin enthaltenen Zellen oder Zellverbänden durch Inkubation des Zellgewebes mit einer Collagenase Class II aus Clostridium histolyticum, welche nach 12 Einfrier-/Auftauzyklen noch in beträchtlichem Umfang Collagenase-Aktivität besitzt und in einem charakteristischen Teilbereich SEQ ID NO:2 enthält oder in diesem Bereich von SEQ ID NO:1 oder einer Sequenz, welche im Rahmen der Degeneration des genetischen Codes für die gleiche Aminosäuresequenz codiert wird und das Produkt einer prokaryontischen oder eukaryontischen Expression einer exogenen DNA ist, bis zur Freisetzung der Zellen oder Zellverbände im gewünschten Umfang und Abtrennung der Zellen oder der Zellverbände von den Zellgewebeanteilen.

2. Verfahren nach Anspruch 1, dadurch gekennzeichnet, daß eine Collagenase Class II verwendet wird, welche die Aminosäuresequenz SEQ ID NO: 10 besitzt oder von SEQ ID NO: 9 oder einer Sequenz, welche im Rahmen der Degeneration des genetischen Codes für die gleiche Aminosäuresequenz codiert.

3. Verfahren nach den Ansprüchen 1 oder 2, dadurch gekennzeichnet, daß als Gewebe Pankreasgewebe verwendet wird, die isolierten Zellen Inselzellen und die Zellverbände Inseln sind.

4. Verwendung einer Collagenase Class II aus Clostridium histolyticum, welche in einem charakteristischen Teilbereich SEQ ID NO:2 enthält oder von SEQ ID NO:1 oder einer Sequenz, welche im Rahmen der Degeneration des genetischen Codes für die gleiche Aminosäuresequenz codiert, codiert wird und das Produkt einer prokaryontischen oder eukaryontischen Expression einer exogenen DNA-Sequenz ist, zur Auflösung von Zellgewebe und Freisetzung von darin enthaltenen Zellen oder Zellverbänden.

5. Verwendung einer Collagenase Class II nach Anspruch 4, dadurch gekennzeichnet, daß eine Collagenase Class II verwendet wird, welche die Aminosäuresequenz SEQ ID NO: 10 besitzt oder von SEQ ID NO: 9 oder einer Sequenz, welche im Rahmen der Degeneration des genetischen Codes für die gleiche Aminosäuresequenz codiert.

6. Verfahren zur Herstellung eines Polypeptids, welches die Eigenschaften einer Collagenase Class II aus Clostridium histolyticum hat und nach 12 Einfrier-/Auftauzyklen noch in beträchtlichem Umfang Collagenase-Aktivität besitzt, durch Expression einer exogenen DNA-Sequenz in prokaryontischen oder eukaryontischen Wirtszellen und Isolierung des gewünschten Polypeptids, wobei die DNA-Sequenz im für diese Collagenase charakteristischen Bereich SEQ ID NO:1 oder eine Sequenz, welche im Rahmen der Degeneration des genetischen Codes für die gleiche Aminosäuresequenz codiert, enthält.

7. Verfahren nach Anspruch 6, dadurch gekennzeichnet, daß die DNA-Sequenz die Teilsequenz SEQ ID NO:1 enthält.

8. Verfahren nach den Ansprüchen 6 oder 7, dadurch gekennzeichnet, daß eine Collagenase Class II verwendet wird, welche die Aminosäuresequenz SEQ ID NO: 10 besitzt oder von SEQ ID NO: 9 oder einer Sequenz, welche im Rahmen der Degeneration des genetischen Codes für die gleiche Aminosäuresequenz codiert.

9. DNA-Sequenz, welche als Teilsequenz SEQ ID NO:1 enthält und für ein Polypeptid mit Collagenase-Aktivität codiert.

10. DNA-Sequenz SEQ ID NO:1.

11. DNA-Sequenz SEQ ID NO: 9.

12. Biologisch funktionelles Plasmid oder viraler DNA-Vektor, der eine DNA-Sequenz nach den Ansprüchen 9 bis 11 enthält.

13. Prokaryontische oder eukaryontische Wirtszelle, welche mit einem DNA-Vektor nach Anspruch 12 stabil transformiert oder transfiziert ist.

14. Polypeptid, welches die Eigenschaften einer Collagenase Class II aus Clostridium histolyticum hat, nach 12 Einfrier-/Auftauzyklen noch in beträchtlichem Umfang Collagenase-Aktivität besitzt und erhältlich ist durch Expression einer exogenen DNA-Sequenz in prokaryontischen oder eukaryontischen Wirtszellen und Isolierung des gewünschten Polypeptids, wobei die DNA-Sequenz im für diese Collagenase charakteristischen Bereich SEQ ID NO:1 oder eine Sequenz, welche im Rahmen der Degeneration des genetischen Codes für die gleiche Aminosäuresequenz codiert, enthält.

15. Polypeptid nach Anspruch 14, dadurch gekennzeichnet, daß es die Proteinsequenz SEQ ID NO: 10 besitzt, oder von SEQ ID NO: 9 codiert wird, oder codiert wird von einer Sequenz, welche im Rahmen der Degeneration des genetischen Codes für die gleiche Aminosäuresequenz codiert.
